# NEUE EUROPÄISCHE PATENTSCHRIFT

(11) **EP 0 428 845 B2**
(45) Veröffentlichungstag und Bekanntmachung des Hinweises auf die Entscheidung über den Einspruch: **23.12.1998**
(45) Hinweis auf die Patenterteilung: 03.11.1993
(21) Anmeldenummer: 90117534.9
(22) Anmeldetag: 12.09.1990
(51) Int. Cl.: B01J 23/50, B01J 23/66, C07D 301/10

(54) **Silberkatalysator für die Oxidation von Ethylen und Verfahren zu seiner Herstellung**
Silver catalyst for the oxidation of ethylene and process for its preparation
Catalyseur à base d'argent pour l'oxydation de l'éthylène et son procédé de préparation

(30) Priorität: 09.11.1989 DE 3937247; 30.03.1990 DE 4010182
(43) Veröffentlichungstag der Anmeldung: 29.05.1991
(73) Patentinhaber: HÜLS AKTIENGESELLSCHAFT, 45764 Marl (DE)
(72) Erfinder: Wunde, Christian, Dr., D-4370 Marl (DE); Kyewski, Dietmar, D-4358 Haltern (DE); Dibowski, Wilma, D-4370 Marl (DE)
(74) Vertreter: Grünecker, Kinkeldey, Stockmair & Schwanhäusser Anwaltssozietät

(56) Entgegenhaltungen:
- EP-A- 271 814
- EP-A- 0 017 725
- EP-A- 0 327 356
- DE-A- 1 221 620
- DE-A- 3 334 347
- FR-A- 2 117 183
- GB-A- 2 117 263
- US-A- 2 424 083
- Chem.Eng.Fund. vol. 2, no.1 (1981) Seiten 23-38
- Journal of Catalysts, vol. 87, (1984), Seiten 116-125

## Beschreibung

Die Erfindung betrifft einen Silberkatalysator für die Oxidation von Ethylen mit Sauerstoff und ein Verfahren zu seiner Herstellung.

Die Erfindung bezweckt, das Verfahren zur Herstellung von Ethylenoxid durch Oxidation von Ethylen wirtschaftlicher zu gestalten.

Bekanntlich werden Silberkatalysatoren zur Herstellung von Ethylenoxid mittels einer gesteuerten unvollständigen Oxidation von Ethylen mit molekularem Sauerstoff eingesetzt. Es sind bereits zahlreiche Modifikationen des Silberkatalysators vorgeschlagen worden. Aus DE-20 17 733, DE-29 14 640 sowie DE-33 34 347 ist bekannt, metallisches Silber aus silberhaltigen Suspensionen oder Gemischen aus Suspensionen und Lösungen auf wenig porösen Katalysatorträgern abzuscheiden. Bei diesen Katalysatoren liegt das Silber erfahrungsgemäß in einer weitgehend zusammenhängenden Schicht mit schwankender Dicke vor, die aus verhältnismäßig großen Silberpartikeln besteht, und die die Oberfläche des Trägers sowie die Wandung der Kanäle und Löcher im Träger bedeckt. Eine derartige Schicht kann leicht mechanisch beschädigt werden; dadurch entsteht Silberstaub im Reaktionsrohr, und die Druckdifferenz zwischen den Enden des Reaktionsrohres steigt während des Betriebes. Infolgedessen sinkt der Gasdurchsatz und die Leistung des Reaktors. Bei derartigen nach dem "Suspensionsverfahren" hergestellten Katalysatoren nimmt die Aktivität und Selektivität besonders schnell ab.

Aus DE-21 59 346 und EP-0 161 930 ist bekannt, Silberkatalysatoren nach dem Imprägnierverfahren herzustellen. Dabei wird ein poröser Katalysatorträger in eine Lösung aus einer oder mehreren Silberverbindungen eingetaucht. Derartige Tränkkatalysatoren zeigen zwar ein günstiges Alterungsverhalten, sie haben jedoch in der Regel am Anfang ihrer Einsatzdauer eine geringere Aktivität und Selektivität als Suspensionskatalysatoren.

Beispielsweise nach DE-23 00 512, DE-27 34 912, EP-0 266 015 und EP-0 229 465 kann man die Selektivität von Tränkkatalysatoren verbessern, indem man Alkalimetall- und andere Promotoren zusätzlich auf dem Träger niederschlägt. Eine zur Verbesserung der Selektivität günstige Dotierung von Promotoren hat jedoch erfahrungsgemäß eine Einbuße in der Aktivität zur Folge. Durch Auswahl von nach Art und Menge geeigneten Promotoren allein läßt sich ein hoch selektiver und gleichzeitig hoch aktiver Katalysator nicht herstellen.

Nach US-2 424 083 wird ein Silber-Katalysator hergestellt mittels naßchemischer Umwandlung eines Silbersalzes - in Gegenwart einer starken Base - zu Silberoxid, welches als Suspension vorliegt und auf dem Katalysatorträger niedergeschlagen wird. Metallisches Silber, das sich dabei bereits gebildet hat, wird zunächst mit Ammoniumhydroxid gelöst. Anschließend wird das auf dem Träger abgeschiedene Silberoxid mit einem Reduktionsmittel zu metallischem Silber reduziert.

In GB-2 117 263 ist ein Verfahren zur Herstellung eines Silberkatalysators angegeben. Auf einem Träger, der fähig ist, Alkalimetalle selektiv zu adsorbieren, wird Silber abgeschieden, welches in Gegenwart von Sauerstoff z. B. Luft, aktiviert wird, damit alle organischen Substanzen vollständig oxidiert werden.

Nach DE-33 21 895 werden Tränkkatalysatoren mit Alkali und Erdalkali-Promotoren in vergleichsweise hoher Konzentration dotiert und anschließend auf Temperaturen von 450 °C bis 800 °C erhitzt, um dadurch die Aktivität zu steigern. Erfahrungsgemäß sind auch bei diesen Katalysatoren Aktivität und Selektivität verbesserungsbedürftig.

Damit stellt sich die Aufgabe, einen gattungsgemäßen Silberkatalysator zu entwickeln, welcher bei gleich großem oder geringerem Silbergehalt im Vergleich zu herkömmlichen Silberkatalysatoren eine verbesserte Aktivität und Selektivität hat.

Diese Aufgabe wird erfindungsgemäß gelöst durch einen Silberkatalysator mit folgenden Merkmalen:
- stückiges homogen poröses hitzbeständiges Material wie z. B. alpha-Aluminiumoxid als Träger mit einer BET-Oberfläche von weniger als 2 m²/g,
- 0,1 bis 25 Massenprozent metallisches Silber, bezogen auf die Masse des fertigen Katalysators, das in feinverteilter Form auf der inneren und äußeren Trägeroberfläche vorliegt.
- eine innerhalb eines Trägerteilchens ungleichförmige Konzentration des Silbers, die auf der äußeren (geometrischen) Oberfläche und in der oberflächennahen Schicht eines Trägerteilchens deutlich größer ist als im Inneren des Trägerteilchens, wobei die oberflächennahe Schicht weniger als 0,5 mm dick ist,
- Silberpartikel, die nach dem thermischen Formieren überwiegend einen Durchmesser von 0,01 bis 1 µm haben, erhältlich durch
- Auswählen solcher mäßig löslichen Silberverbindungen und Formulierungen, die bei 50°C eine Lösung mit weniger als 400 g Silber pro Liter Lösung ergeben,
- Einstellen der Silberkonzentration der Imprägnierlösung auf einen Wert, bei dem die Konzentration der Silberverbindung mindestens 10% unter der Sättigungskonzentration liegt,
- Einstellen der Temperatur beim Imprägnieren des Trägers auf einen Wert, bei der die Silberverbindung in Lösung bleibt,
- Vortrortrocknen des imprägnierten Trägers bei einer Temperatur von 40°C bis 120°C,
- thermisches Formieren des Trägers in einem Gasstrom,
wobei während des Imprägnierens und Vortrocknens wiederhold evakuiert wird.

Die unter der geometrischen Oberfläche des Trägerteilchens liegende Schicht mit deutlich größerer Konzentration des Silbers hat eine Dicke von bevorzugt weniger als 0,3 mm.

Der Katalysator kann außer Silber noch Natrium-, Kalium, Rubidium, Cäsium- und/oder Bariumverbindungen enthalten, bevorzugt in einer Menge von 0,0005 bis 0,03 Grammäquivalent pro Kilogramm Katalysator.

Der poröse hitzebeständige Träger besteht bevorzugt aus alpha-Aluminiumoxid in einer Reinheit von mehr als 80 Massen-%.

Der erfindungsgemäße Katalysator wird hergestellt nach einem Verfahren mit den folgenden Merkmalen:
- Auswählen solcher, mäßig löslicher Silberverbindungen und Formulierungen, die bei 50 °C eine Lösung mit weniger als 400 g Silber
- bevorzugt weniger als 330 g Silber - pro Liter Lösung ergeben,
- Einstellen der Silberkonzentration der Imprägnierlösung auf einen Wert, bei dem die Konzentration der Silberverbindung mindestens 10 % - bevorzugt mindestens 20 % - unter der Sättigungskonzentration beim Imprägnieren liegt,
- Einstellen der Temperatur beim Imprägnieren des Trägers auf einen Wert, bei dem die Silberverbindung in Lösung bleibt,
- Vortrocknen des imprägnierten Trägers bei einer Temperatur von 40 °C bis 120 °C, bevorzugt von 60 °C bis 90 °C,
- thermisches Formieren des Trägers in einem Gasstrom,
wobei während des Imprägnierens und Vortrocknens wiederholt evakuiert wird.

Als Lösemittel für die silberhaltige Lösung dient Wasser oder eine - ggf. wasserhaltige - organische Flüssigkeit, wie z. B. Ethanol, oder ein Gemisch von - ggf. wasserhaltigen - organischen Flüssigkeiten.

Anstatt eine Silberverbindung auszuwählen, kann auch eine - gegebenenfalls handelsübliche - kolloidale Silberlösung eingesetzt werden, wobei das Silberkolloid entweder ungeschützt oder z. B. durch ein anderes Kolloid geschützt vorliegen kann.

Der Katalysator-Träger wird mit der silberhaltigen Lösung imprägniert und in einer bewegten oder ruhenden Schüttung vorgetrocknet.

Das Gefäß, das den imprägnierten Träger enthält, wird während des Imprägnierens und Vortrocknens wiederholt evakuiert. Beim Vortrocknen liegt der Druck oberhalb 20 kPa (200 mbar) absolut. Das Vortrocknen dauert mindestens 30 Minuten, bevorzugt mindestens 60 Minuten. Das Imprägnier- und Trocknungsgefäß wird wiederholt belüftet und evakuiert, und zwar in Abständen von 1 bis 30 Minuten - bevorzugt 5 bis 15 Minuten - während einer jeweiligen Dauer von 1 bis 30 Minuten - bevorzugt 3 bis 15 Minuten. Nach dem Vortrocknen wird der Träger in einem Gasstrom bei 50 °C bis 150 °C weiter getrocknet und schließlich - wie üblich - thermisch in einer dünnen bewegten Schicht formiert, bevorzugt während 5 bis 240 Minuten bei einer Temperatur des Formiergases von 100 °C bis 250 °C. Das Formiergas besteht bevorzugt überwiegend aus Stickstoff und enthält maximal 12 % Sauerstoff. Die Trägerteilchen liegen beim Formieren in einer bewegten Schicht mit einer Schichtdicke, die dem Ein- bis Fünffachen der kleinsten Abmessung eines Trägerteilchens entspricht.

Geeignete poröse Träger für die erfindungsgemäßen Katalysatoren sind handelsübliche alpha-Aluminiumoxide mit einer BET-Oberfläche von weniger als 2 m²/g und einem Teilchendurchmesser von 5 bis 10 mm. Die Trägerteilchen können ringförmig, kugelförmig oder anders geformt sein.

Das Tränkverfahren kann ein- oder mehrstufig sein. Die Promotor-Verbindungen können gleichzeitig mit den Silberverbindungen oder getrennt davon abgeschieden werden.

Wie sich überraschenderweise gezeigt hat, hat ein Silberkatalysator, bei dem das Silber in einer dünnen Schicht an und unter der geometrischen Oberfläche der Trägerteilchen angereichert ist, günstige Eigenschaften. Einen derartigen Katalysator erhält man, wenn man beim Imprägnieren der Trägerteilchen mit der silberhaltigen Lösung und beim Vortrocknen der imprägnierten Trägerteilchen Verfahrensbedingungen einhält, die das Abscheiden der Silberverbindungen oder das Abscheiden von metallischem Silber an der geometrischen Oberfläche und in der oberflächennahen Schicht des porösen Trägers begünstigen. Dabei sind die Löslichkeit der Silberverbindung in dem gewählten Lösemittel, die Viskosität der Lösung oder der Suspension und gegebenenfalls die Größe der kolloidalen Teilchen und ihr elektrisches Grenzflächenverhalten bei der während des Imprägnierens und während des Vortrocknens vorliegenden Temperatur zu berücksichtigen.

Außerdem sind beim Vortrocknen und beim Formieren zu beachten:
- Die Temperaturen - wobei Start- und Endtemperatur unterschiedlich sein können - unter Berücksichtigung der jeweils eingesetzten Silberverbindung,
- die Belüftung des teilevakuierten Imprägnier- und Trocknungsgefäßes in optimierten Zeitspannen und Zeitabständen unter Berücksichtigung der Silberverbindungen, des Lösemittels, der Behälterund Ansatzgröße sowie der Heizung des Behälters,
- der Enddruck beim Vortrocknen unter Unterdruck,
- die Steuerung des Druckes, die die Zeitspanne des Trocknungsvorganges unter vermindertem Druck bestimmt,
- die Gaszusammensetzung, Gastemperatur und Strömungsgeschwindigkeit beim Trocknen und Formieren im Gasstrom,
- die Dicke der Katalysatorschicht beim Trocknen und Formieren,
- die jeweiligen Verweilzeiten der Trägerteilchen während der verschiedenen Phasen dieser Behandlungsvorgänge.

Die verstärkte Abscheidung von Silberverbindungen in der oberflächennahen Schicht der porösen Trägerteilchen wird unter anderem durch Chromatographieeffekte bewirkt. Sie wird ferner durch Initiatoren, z. B. Bariumperoxid, beeinflußt, die die Entstehung von Kristallkeimen begünstigen.

Die Verteilung des Silbers innerhalb der Trägerteilchen des fertigen Katalysators läßt sich mittels eines Raster-Elektronenmikroskops mit Einrichtung zur energiedispersiven Röntgenanalyse (EDX) ermitteln.

Der erfindungsgemäße Katalysator hat folgende Vorteile:
- Die Aktivität des Katalysators ist deutlich höher im Vergleich zur Aktivität gattungsgemäßer Katalysatoren, deren Silbergehalt gleich groß oder etwas höher ist, bei unveränderter oder etwas erhöhter Selektivität. Der Katalysator kann bei einer niedrigeren Betriebstemperatur eingesetzt werden.
- Der Katalysator altert langsamer als ein vergleichbarer Katalysator nach dem Stand der Technik. Dadurch wird seine Standzeit größer und die zur Herstellung einer bestimmten Menge Ethylenoxid benötigte Silbermenge wird kleiner.
- Durch die ungleichmäßige Verteilung des Silbern innerhalb des Trägerteilchens und die Anreicherung in einer dünnen Schicht an und unterhalb der geometrischen Oberfläche des Trägers wird der Stoffund Wärmetransport während der bestimmungsgemäßen Gasphasenreaktion begünstigt, und die Reaktion läßt sich zweckmäßiger führen. Bei Katalysatoren nach dem Stand der Technik, bei denen das Silber innerhalb des porösen Trägers in etwa gleichmäßig verteilt ist, liefern die in tieferen Schichten des Katalysators befindlichen Silberteilchen generell nureinen relativ geringen Beitrag zur partiellen Oxidation von Ethylen zu Ethylenoxid, und die innerhalb der Trägerteilchen freigesetzte Reaktionswärme wird durch Gastransport generell langsamer abgeführt als die Reaktionswärme, die an der Oberfläche des Katalysatorträgers freigesetzt wird.
- Innerhalb der porösen Trägerteilchen des erfindungsgemäßen Katalysators entsteht noch weniger Ethylenoxid als bei einem Katalysator nach dem Stand der Technik. Dadurch wird der Anteil des Ethylenoxids, der zu Acetaldehyd isomerisiert oder zu Kohlendioxid und Wasser weiter oxidiert wird, kleiner und damit die Selektivität etwas größer.
- Die in den Reaktionsrohren während des Betriebes entstehende Menge an Katalysatorstaub ist geringer als bei herkömmlichen Katalysatoren, die nach dem Suspensionsverfahren hergestellt werden.
- Wegen der niedrigeren Betriebstemperatur kann der Sauerstoffgehalt im Kreisgas erhöht werden, was sich auf Selektivität und Alterungsverhalten des Katalysators günstig auswirkt.

Der erfindungsgemäße Katalysator und das Herstellungsverfahren werden anhand der folgenden Beispiele weiter erläutert.

Für die Herstellung von erfindungsgemäßen - und zum Vergleich nach dem Stand der Technik hergestellten - Katalysatoren wurden verschiedene handelsübliche Träger aus alpha-Aluminiumoxid mit unterschiedlicher chemischer Zusammensetzung und unterschiedlichen physikalischen Eigenschaften sowie unterschiedlicher Teilchengröße und -form verwendet; diese Träger sind in der Tabelle 1 zusamengestellt.

**Tabelle 1**

| | Träger A | Träger B | Träger C | Träger D | |
|---|---|---|---|---|---|
| Gehalt an Aluminiumoxid | 95 | 95 | 86 | 97 | % |
| Teilchendurchmesser | 6,2 | 8,5 | 8,5 | 7,2 | mm |
| Teilchenform | ringförmig | ringförmig | kugelförmig | ringförmig | |
| spez. Oberfläche | 0,35 | 0,35 | 0,29 | 0,43 | m²/g |
| Porenvolumen | 0,39 | 0,43 | 0,35 | 0,41 | cm³/g |
| Schüttgewicht | 758 | 695 | 825 | 747 | g/l |
| Wasserabsorption | 37 | 39 | 41 | 44 | % |
| mittlerer Porendurchm. | 5,2 | 8,6 | - | 3 | µm |

Falls beim Imprägnieren eines Trägers mit (kolloidalen) Lösungen, die das Silber in geringer Konzentration enthalten, ein einmaliges Tränken nicht ausreicht, ist ein mehrfaches Tränken notwendig.

### Beispiel 1

413 g Silbernitrat werden in 2 l Wasser gelöst und mit 25prozentiger wäßriger Natronlauge zu Silberoxid umgesetzt. Das Silberoxid wird mit einer Nutsche von der Natronlauge abgetrennt, mit Wasser alkalifrei gewaschen, in ein Gefäß überführt und in 750 ml Wasser suspendiert. Mit 315g auf 70 °C vorgewärmter Milchsäure wird eine Silberlactatlösung hergestellt die mit 0,55 g Bariumperoxid versetzt und mit Wasser auf 1 500 ml verdünnt wird.

Mit dieser Silberlactatlösung werden 3,3 l des Trägers A (siehe Tabelle 1) in einem Rotationsverdampfer imprägniert. Der vom Träger nicht aufgenommene Rest der Imprägnierlösung verbleibt im Kolben des Rotationsverdampfers. Die Badtemperatur des Rotationsverdampfers wird auf 70 °C eingestellt, und das Lösemittel wird bei Unterdruck abgezogen. Alle 7 Minuten wird der Kolben belüftet und 5 Minuten unter Normaldruck gehalten. Nach 3 bis 4 Stunden erreicht man im Kolben einen Druck von 20 kPa (200 mbar).

Der imprägnierte und vorgetrocknete Träger wird auf einer elektrisch beheizten Siebbandmaschine zunächst bei einer eingestellten Temperatur von 80 °C und anschließend bei 120 °C getrocknet. Die Siebbandmaschine hat eine Länge von 3,2 m; bei einer Bandgeschwindigkeit von ca. 6 cm/Minute beträgt die Verweilzeit ca. 55 Minuten. Die Trägerteilchen liegen auf dem Band in einer Schicht, deren Dicke etwa dem doppelten Teilchendurchmesser entspricht.

Anschließend werden die Trägerteilchen auf der Siebbandmaschine während 55 Minuten bei 180 °C in einem Stickstoff-Luft-Gemisch, das ca. 7 % Sauerstoff enthält, formiert; dabei zersetzt sich die Silberverbindung.

Nun wird, wie oben beschrieben, eine zweite Silberlactatlösung hergestellt, welcher zusätzlich 1,41 g Cäsiumnitrat beigemischt werden. Mit dieser Lösung werden die bereits einmal imprägnierten und formierten Trägerteilchen erneut imprägniert und nochmals vorgetrocknet, jedoch jetzt 55 Minuten lang bei 240 °C auf der Siebbandmaschine nochmals formiert.

Auf der äußeren Oberfläche der Trägerteilchen des fertigen Katalysators und in der etwa 0,2 mm dicken oberflächennahen Schicht ist das Silber - im Vergleich zum Inneren der Trägerteilchen - deutlich angereichert.

### Beispiel 2

3,3 l des Trägers B (siehe Tabelle 1) werden analog zu Beispiel 1 imprägniert, vorgetrocknet und formiert.

Die Silberlösung für das zweite Imprägnieren wird mit 1,17 g Cäsiumnitrat und 0,55 g Bariumperoxid versetzt. Hiermit wird der Träger zum zweiten Mal imprägniert und analog zu Beispiel 1 vorgetrocknet und formiert.

Auf der äußeren Oberfläche der Trägerteilchen des fertigen Katalysators und in der etwa 0,2 mm dicken oberflächennahen Schicht ist das Silber - im Vergleich zum Inneren der Trägerteilchen - deutlich angereichert.

### Beispiel 3

3,3 l des Trägers C (siehe Tabelle 1) werden analog zu Beispiel 1 imprägniert, vorgetrocknet und formiert.

Die Silberlösung für das zweite Imprägnieren wird mit 0,99 g Cäsiumnitrat, 3,0g Natriumnitrat und 0,55g Bariumperoxid versetzt. Hiermit wird der Träger zum zweiten Mal imprägniert und analog zu Beispiel 1 vorgetrocknet und formiert.

Auf der Oberfläche der Trägerteilchen des fertigen Katalysators und in der etwa 0,2 mm dicken oberflächennahen Schicht ist das Silber - im Vergleich zum Kern der Trägerteilchen - deutlich angereichert.

### Beispiel 4

Aus 826 g Silbernitrat, 630 g Milchsäure und 1,1 g Bariumperoxid wird analog zu Beispiel 1 eine Silberlactatlösung mit entsprechendem Volumen hergestellt. Diese Lösung wird mit Wasser auf 5,5 l verdünnt und auf 70 °C erwärmt.

6,6 l des Trägers D (siehe Tabelle 1) werden in ein ca. 8 l fassendes zylindrisches Gefäß mit Mantelbeheizung eingefüllt; die Mantelbeheizung wird auf 70 °C eingestellt. Der Träger wird mit der 70 °C warmen Silberlactatlösung übergossen; für ca. 5 Minuten wird ein geringer Unterdruck eingestellt. Nach dem Belüften wird der vom Träger nicht aufgenommene Teil der Lösung wird abgelassen. Der Träger wird in dieses Gefäß etwa eine Stunde lang bei 70 °C und Unterdruck vorgetrocknet. Der Unterdruck wird dabei zweimal für ca. 7 Minuten aufgehoben.

Mit dem 70 °C warmen verbliebenen Teil der Lösung wird der Träger noch zweimal übergossen und jeweils anschließend in der beschriebenen Weise vorgetrocknet.

Der dreimal übergossene und vorgetrocknete Träger wird in einem Glasgefäß 12 h lang bei 80 °C mittels eines Stickstoffstromes getrocknet und anschließend, analog zu Beispiel 1, auf der Siebbandmaschine 55 Minuten lang bei 180 °C formiert.

Weiter wird aus 826 g Silbernitrat, 630 g Milchsäure sowie 2,3 g Cäsiumnitrat und 1,1 g Bariumperoxid eine Lösung hergestellt und mit Wasser auf 5,5 l verdünnt.

Mit dieser Lösung wird der bereits formierte Träger ein weiteres Mal übergossen, vorgetrocknet und auf der Siebbandmaschine während 55 Minuten bei 240 °C erneut formiert.

Auf der äußeren Oberfläche der Trägerteilchen des fertigen Katalysators und in der etwa 0,3 mm dicken oberflächennahen Schicht ist das Silber - im Vergleich zum Inneren der Trägerteilchen - deutlich angereichert.

### Beispiel 5

Aus 45 g Silbernitrat und 34,3 g Milchsäure wird analog zu Beispiel 1 eine Silberlactatlösung hergestellt und ait Wasser auf 125 ml verdünnt. Zu dieser Lösung werden 25 g Ethanol, 0,10g Cäsiumnitrat und 0,11 g Bariumperoxid hinzugegeben und intensiv verrührt.

0,33 l des Trogers A (siehe Tabelle 1) werden mit dieser Lösung analog zu Beispiel 1 imprägniert, vorgetrocknet und auf der Siebbandmaschine während 55 Minuten bei 180 °C formiert.

Auf der äußeren Oberfläche der Trägerteilchen des fertigen Katalysators und in der etwa 0,2 mm dicken oberflächennahen Schicht ist das Silber- im Vergleich zum Inneren der Trägerteilchen - deutlich angereichert.

### Beispiel 6

Aus 10,3 g Silbernitrat und 7,9 g Milchsäure wird analog zu Beispiel 1 eine Silberlactallösung hergestellt und mit Wasser auf 120 ml verdünnt. Zu dieser Lösung werden 0,11 g Bariumperoxid hinzugegeben und intensiv verrührt.

0,33 l des Trägers A (siehe Tabelle 1) werden mit einerderartigen Lösung analog zu Beispiel 1 imprägniert, vorgetrocknet und formiert, und zwar insgesamt viermal.

Bei diesen mit vier Silberaufzügen versehenen Trägerteilchen des fertigen Katalysators ist das Silber auf der äußeren Oberfläche und in der etwa 0,3 mm dicken oberflächennahen Schicht- im Vergleich zum Inneren der Trägerteilchen - besonders deutlich angereichert.

### Beispiel 7

Aus 20,6g Silbernitrat und 16,5g Milchsäure wird analog zu Beispiel 1 eine Silberlactatlösung hergestellt und mit Wasser auf 50 ml verdünnt.

Weitere 20,6 g Silbernitratwerden analog zu Beispiel 1 in eine Silberoxid-Suspension überführt und durch Zugeben einer Lösung von 5,5 g Malonsäure in 15 g Wasser in eine Silbermalonat-Suspension überführt.

Beide silberhaltigen Flüssigkeiten werden vereinigt; unter Rühren und Kühlen wird eine Mischung aus 18,3 g Isopropylamin und 18,3 g t-Butylamin langsam zugetropft. Diese Lösung wird mit Wasser auf 150 ml verdünnt und mit 0,11 g Bariumperoxid versetzt.

0,33 l des Trägers A (siehe Tabelle 1) werden mit dieser Lösung analog zu Beispiel 1 imprägniert, vorgetrocknet und formiert.

Auf der äußeren Oberfläche der Trägerteilchen des fertigen Katalysators und in einer etwa 0,2 mm dicken oberflächennahen Schicht ist das Silber- im Vergleich zum Inneren der Trägerteilchen - deutlich angereichert.

### Beispiel 8

Aus 45g Silbernitrat wird analog zu Beispiel 1 eine Silberoxid-Suspension und anschließend mit 17,5g Oxalsäure-Dihydrat eine wäßrige Silberoxalat-Suspension hergestellt. Diese wird mit einer Mischung aus 20 g Isopropylamin und 20g t-Butylamin unter Rühren und Kühlen in eine klare Lösung überführt, und unter Rühren werden 0,11 g Bariumperoxid hinzugegeben. Diese Lösung wird mit Wasser auf 150 ml verdünnt.

0,33 l des Trägers A (siehe Tabelle 1) werden analog zu Beispiel 1 mit dieser Lösung imprägniert, vorgetrocknet und formiert.

Auf der äußeren Oberfläche der Trägerteilchen des fertigen Katalysators und in der etwa 0,1 mm dicken oberflächennahen Schicht ist das Silber - im Vergleich zum Inneren der Trägerteilchen - sehr deutlich angereichert.

### Beispiel 9

Aus 45g Silbernitrat wird analog zu Beispiel 1 eine Silberoxid-Suspension hergestellt. Das Silberoxid wird mit 200 g Trockeneis zu Silbercarbonat umgesetzt, dieses mit einem Gemisch aus 26 g Isopropylamin und 26 g t-Butylamin unter Rühren und Kühlen zu den entsprechenden Silberamin-Komplexen umgesetzt, mit Wasser auf 150 ml verdünnt und mit 0,11 g Bariumperoxid innig vermischt.

0,33 l des Trägers A (siehe Tabelle 1) werden mit der auf 70 °C vorgewärmten Lösung imprägniert, vorgetrocknet und analog zu Beispiel 1 bei 180 °C formiert.

Auf der äußeren Oberfläche der Trägerteilchen des fertigen Katalysators und in einer etwa 0,1 mm dicken oberflächennahen Schicht ist das Silber - im Vergleich zum Inneren der Trägerteilchen - sehr deutlich angereichert.

### Beispiel 10

Mit 100 ml einer kolloidalen Silberlösung (Hersteller Degussa; 0,8% Silber in wäßriger Lösung mit Schutzkolloid (Poly-oxycarbonsäure) werden 37 g des Trägers A (siehe Tabelle 1) in einem Rotationsverdampfer bei Unterdruck und Raumtemperatur imprägniert; innerhalb von 10 Minuten wird der Kolben zweimal belüftet und 1 Minute unter Normaldruck gehalten. Die vom Katalysatorträger nicht aufgenommene Restlösung wird abdekantiert. Das Lösemittel wird bei Unterdruck abgezogen. Die Badtemperatur wird innerhalb von 10 Minuten von Raumtemperatur auf 95 °C angehoben. Anschließend wird der Kolben alle 7 Minuten belüftet und 5 Minuten unter Normaldruck gehalten. Nach ca. 35 Minuten erreicht man im Kolben einen Druck von 20 kPa (200 mbar).

Der imprägnierte und vorgetrocknete Träger wird auf einer elektrisch beheizten Siebbandmaschine zunächst bei einer eingestellten Temperatur von 105 °C in einem Stickstoff-Luft-Gemisch mit ca. 7 % Sauerstoff getrocknet. Die Siebbandmaschine hat eine Länge von 3,2 m; bei einer Bandgeschwindigkeit von ca. 6 cm/Minute beträgt die Verweilzeit ca. 55 Minuten. Die Trägerteilchen liegen auf dem Band in einer Schicht, deren Dicke etwa dem doppelten Teilchendurchmesser entspricht.

Anschließend werden die Teilchen auf der Siebbandmaschine während 55 Minuten bei 200 °C in einem Stickstoff-Luft-Gemisch, das ca. 7 % Sauerstoff enthält, formiert.

Die Teilchen werden, wie oben beschrieben, noch zweimal imprägniert, im Rotationsverdampfer vorgetrocknet und auf der Siebbandmaschine getrocknet und formiert, wie oben beschrieben.

Auf der äußeren Oberfläche der Trägerteilchen des fertigen Katalysators und in der etwa 0,2 mm dicken oberflächennahen Schicht ist das Silber - im Vergleich zum Inneren der Trägerteilchen - sehr deutlich angereichert. Die mittleren Bereiche der Trägerteilchen sind fastfrei von Silber. Der Durchmesser der Silberpartikel liegt überwiegend unterhalb 0,1 µm.

### Beispiel 11

Mit 20 ml einer kolloidalen Silberlösung (siehe Beispiel 10) werden 37 g des Trägers A (siehe Tabelle 1) in einem Rotationsverdampfer imprägniert. Der vom Träger nicht aufgenommene Rest der Imprägnierlösung verbleibt im Kolben des Rotationsverdampfers. Das Bad des Rotationsverdampfers hatwährend der ersten 10 Minuten Raumtemperatur. Anschließend wird die Badtemperatur innerhalb ca. 10 Minuten auf 95 °C angehoben. Das Lösemittel wird bei Unterdruck abgezogen. Der Kolben wird alle 7 Minuten belüftet und 5 Minuten unter Normaldruck gehalten. Nach ca. 50 Minuten erreicht man im Kolben einen Druck von 20 kPa (200 mbar).

Der imprägnierte und vorgetrocknete Trägerwird aufeinerelektrisch beheizten Siebbandmaschine bei 105 °C und anschließend bei 200 °C in einem Gasstrom eines Stickstoff-Luft-Gemisches, welches ca. 7 % Sauerstoff enthält, getrocknet und formiert, wie in Beispiel 10 beschrieben.

Nun werden die Katalysatorteilchen ein weiteres Mal imprägniert, im Rotationsverdampfer vorgetrocknet und auf der Siebbandmaschine getrocknet und formiert, wie oben beschrieben.

Auf der äußeren Oberfläche der Trägerteilchen des fertigen Katalysators und in der etwa 0,2 mm dicken oberflächennahen Schicht ist das Silber - im Vergleich zum Inneren der Trägerteilchen - sehr deutlich angereichert. Die mittleren Bereiche der Trägerteilchen enthalten nurwenige vereinzelte Silberteilchen. Der Durchmesser der Silberteilchen liegt überwiegend unterhalb 0,1 µm.

### Beispiel 12

Mit 20 ml einer kolloidalen Silberlösung (siehe Beispiel 10) werden 37 g des Trägers B (siehe Tabelle 1) in einem Rotationsverdampfer- wie in Beispiel 11 beschrieben - imprägniert und vorgetrocknet und auf einer Siebbandmaschine - analog zu den Beispielen 10 und 11 - getrocknet und formiert. Nach dergleichen Methode schließt sich eine weitere Imprägnierung mit Vortrocknen, Trocknen und Formieren an.

Auf der äußeren Oberfläche der Trägerteilchen des fertigen Katalysators und in der etwa 0,2 mm dicken oberflächennahen Schicht ist das Silber - im Vergleich zum Inneren der Trägerteilchen - sehr deutlich angereichert. Die mittleren Bereiche der Trägerteilchen enthalten nurwenige vereinzelte Silberteilchen. Der Durchmesser der Silberteilchen liegt überwiegend unterhalb 0,1 µm.

### Beispiel 13

400 ml einer kolloidalen Silberlösung (siehe Beispiel 10) werden im Rotationsverdampfer unter vermindertem Druck bis auf 100 ml eingeengt.

37 g des Trägers B (siehe Tabelle 1) werden - entsprechend dem in Beispiel 11 angegebenen Verfahren - zweimal mit je 20 ml der eingeengten kolloidalen Silberlösung imprägniert, vorgetrocknet, getrocknet und formiert, wie oben beschrieben.

Auf der äußeren Oberfläche der Trägerteilchen des fertigen Katalysators und in der etwa 0,2 mm dicken oberflächennahen Schicht ist das Silber - im Vergleich zum Inneren der Trägerteilchen - sehr deutlich angereichert. Der Durchmesser der Silberpartikel liegt überwiegend unterhalb 0,1 µm.

Wegen der Neigung einiger aminhaltiger Silberformulierungen, sich im vorgetrockneten Zustand ggf. sehr heftig zu zersetzen, sind die aminfreien Formulierungen vorzuziehen.

Die erfindungsgemäßen und vergleichsweise nach dem Stand der Technik hergestellten Katalysatoren werden in einer Apparatur geprüft, die aus einem Reaktionsrohr von 6 m Länge und 26 mm Durchmesser aus rostfreiem Stahl besteht. Das Reaktionsrohr ist von einem Mantel umgeben, der Wasser oder Wasserdampf zum Abführen der Reaktionswärme enthält. Das Reaktionsrohr wird jeweils mit 2,7 l fertigem Katalysator gefüllt.

Eine Gasmischung, bestehend aus:
30 Vol-% C₂H₄
50 Vol-% CH₄
6,8 Vol-% O₂
4,0 Vol-% CO₂
0,3 Vol-% C₂H₆
Rest Ar und N₂
wird mit einem Durchsatz von 20 Nm³/h über den Katalysator geleitet. Der Druck im Reaktor beträgt 1,9 MPa (19 bar) Überdruck. Der Gasmischung wird ein chlorhaltiger Moderator zugegeben, so daß der Gesamtchlorgehalt im Reaktionsgas bei 8 mg Chlor/Nm³ liegt.

Die erfindungsgemäßen in den Beispielen beschriebenen fertigen Katalysatoren, bei denen das Silber auf der geometrischen Oberfläche und in der oberflächennahen Schicht deutlich angereichert ist, zeigen im direkten Vergleich zu den ihnen entsprechenden bekannten fertigen Katalysatoren, die ausjeweils dem gleichen Träger und mit jeweils dem gleichen Silbergehalt und den gleichen Dotierungen hergestellt wurden, aber eine über den gesamten Querschnitt der Trägerteilchen gleichmäßige Silberverteilung aufweisen, Aktivitätsverbesserungen, die einer Absenkung der Reaktionstemperatur von 5 bis 10 Grad entsprechen, und Selektivitätsverbesserungen von 0,3 bis 1,0 % bei jeweils der gleichen Produktionsrate von Ethylenoxid.

## Patentansprüche

1. Silberkatalysator für die Oxidation von Ethylen zu Ethylenoxid mit einem Gehalt von 0,1 bis 25 Massenprozent Silber - bezogen auf die Masse des fertigen Katalysators - auf einem homogen porösen hitzebeständigen Träger mit einer BET-Oberfläche von weniger als 2 m²/g, einer innerhalb eines Trägerteilchens ungleichförmigen Konzentration des Silbers, die auf der geometrischen Oberfläche und in der oberflächennahen Schicht eines Trägerteilchens, die weniger als 0,5 mm dick ist, deutlich größer ist als im Inneren des Trägerteilchens, wobei die Silberpartikel überwiegend einen Durchmesser von 0,01 bis 1 µm haben, erhältlich durch
- Auswählen solcher mäßig löslichen Silberverbindungen und Formulierungen, die bei 50°C eine Lösung mit weniger als 400 g Silber pro Liter Lösung ergeben,
- Einstellen der Silberkonzentration der Imprägnierlösung auf einen Wert, bei dem die Konzentration der Silberverbindung mindestens 10% unter der Sättigungskonzentration liegt,
- Einstellen der Temperatur beim Imprägnieren des Trägers auf einen Wert, bei der die Silberverbindung in Lösung bleibt,
- Vortrocknen des imprägnierten Trägers bei einer Temperatur von 40°C bis 120°C,
- thermisches Formieren des Trägers in einem Gasstrom,
wobei während des Imprägnierens und Vortrocknens wiederholt evakuiert wird.

2. Silberkatalysator nach Anspruch 1, gekennzeichnet durch
- eine oberflächennahe Schicht des Trägerteilchens, die weniger als 0,3 mm dick ist, mit deutlich größerer Konzentration des Silbers.

3. Silberkatalysator nach den Ansprüchen 1 bis 2,
gekennzeichnet durch
- zusätzlich zum Silber vorhandene Promotoren, bestehend aus Natrium-, Kalium-, Rubidium-, Cäsium- und/oder Bariumverbindungen in einer Konzentration von 0,0005 bis 0,03 Grammäquivalent pro Kilogramm Katalysator.

4. Silberkatalysator nach Anspruch 1 bis 3,
gekennzeichnet durch
- einen porösen hitzebeständigen Träger, der aus alpha-Aluminiumoxid in einer Reinheit von mehr als 80 Massenprozent besteht.

5. Verfahren zur Herstellung eines Silberkatalysators nach den Ansprüchen 1 bis 4, bei dem ein homogen poröser hitzebeständiger Träger mit einer silberhaltigen Lösung imprägniert wird und der imprägnierte Träger vorgetrocknet und thermisch formiert wird,
gekennzeichnet durch
- Auswählen solcher mäßig löslichen Silberverbindungen und Formulierungen, die bei 50°C eine Lösung mit weniger als 400 g Silber pro Liter Lösung ergeben,
- Einstellen der Silberkonzentration der Imprägnierlösung auf einen Wert, bei dem die Konzentration der Silberverbindung mindestens 10% unter der Sättigungskonzentration liegt,
- Einstellen der Temperatur beim Imprägnieren des Trägers auf einen Wert, bei der die Silberverbindung in Lösung bleibt,
- Vortrocknen des imprägnierten Trägers bei einer Temperatur von 40°C bis 120°C,
- thermisches Formieren des Trägers in einem Gasstrom,
wobei während des Imprägnierens und Vortrocknens wiederholt evakuiert wird.

6. Verfahren nach Anspruch 5, gekennzeichnet durch
- Auswählen solcher mäßig löslichen Silberverbindungen und Formulierungen, die bei 50°C eine Lösung mit weniger als 330 g Silber pro Liter Lösung ergeben,
- Einstellen der Silberkonzentration der Imprägnierlösung auf einen Wert, bei dem die Konzentration der Silberverbindung mindestens 20% unter der Sättigungskonzentration liegt,
- Einstellen der Temperatur beim Imprägnieren des Trägers auf einen Wert, bei der die Silberverbindung in Lösung bleibt,
- Vortrocknen des imprägnierten Trägers bei einer Temperatur von 60°C bis 90°C,
- thermisches Formieren des Trägers in einem Gasstrom.

7. Verfahren nach den Ansprüchen 5 und 6, gekennzeichnet durch
- Verwenden von Wasser, oder
- Verwenden einer organischen Flüssigkeit, oder
- Verwenden eines Gemisches von organischen Flüssigkeiten als Lösemittel für die silberhaltige Imprägnierlösung.

8. Verfahren nach den Ansprüchen 5 und 6, gekennzeichnet durch
- Verwenden einer wasserhaltigen organischen Flüssigkeit als Lösemittel für die silberhaltige Imprägnierlösung.

9. Verfahren nach den Ansprüchen 5 und 6, gekennzeichnet durch
- Verwenden von Ethanol als Lösemittel für die silberhaltige Imprägnierlösung.

10. Verfahren nach den Ansprüchen 5 bis 9, gekennzeichnet durch
- Auswählen einer Silberlösung, bei der das Silber in kolloidaler Form vorliegt.

11. Verfahren nach den Ansprüchen 5 bis 10, gekennzeichnet durch
- Auswählen einer kolloidalen Silberlösung, bei der das Silberkolloid durch ein anderes Kolloid geschützt ist.

12. Verfahren nach den Ansprüchen 5 bis 11, gekennzeichnet durch
- Vortrocknen des imprägnierten Trägers mittels eines durch- oder übergeleiteten Gasstromes.

13. Verfahren nach den Ansprüchen 5 bis 12, gekennzeichnet durch
- Vortrocknen des imprägnierten Trägers bei einem verminderten Druck, der oberhalb 20 kPa (200 mbar) absolut liegt, während mindestens 30 Minuten.

14. Verfahren nach den Ansprüchen 5 bis 12, gekennzeichnet durch
- Vortrocknen des imprägnierten Trägers bei einem verminderten Druck, der oberhalb 20 kPa (200 mbar) absolut liegt, während mindestens 60 Minuten.

15. Verfahren nach den Ansprüchen 5 bis 14, gekennzeichnet durch
- wiederholtes Belüften und Evakuieren des Imprägnier- und Trocknungsgefäßes in Abständen von 1 bis 30 Minuten während einer jeweiligen Dauer von 1 bis 30 Minuten.

16. Verfahren nach den Ansprüchen 5 bis 14, gekennzeichnet durch
- wiederholtes Belüften und Evakuieren des Imprägnier- und Trocknungsgefäßes in Abständen von 5 bis 15 Minuten während einer jeweiligen Dauer von 3 bis 15 Minuten.

17. Verfahren nach den Ansprüchen 5 bis 16, gekennzeichnet durch
- Zugeben einer geringen Menge von Initiatoren, die die Bildung von Kristallkeimen begünstigen, zu der Imprägnierlösung.

18. Verfahren nach den Ansprüchen 5 bis 16, gekennzeichnet durch
- Zugeben von Bariumperoxid zu der Imprägnierlösung.

19. Verfahren nach den Ansprüchen 5 bis 18, gekennzeichnet durch
- Formieren der vorgetrockneten Trägerteilchen in einer bewegten Schicht mit einer Schichtdicke, die dem Ein- bis Fünffachen der kleinsten Abmessungen eines Trägerteilchens entspricht, mittels eines Gasstroms, der überwiegend aus Stickstoff mit maximal 12 Massenprozent Sauerstoff besteht, während 5 bis 240 Minuten bei einer Temperatur von 100 bis 250°C.

20. Verfahren nach den Ansprüchen 5 bis 19, gekennzeichnet durch
- Abscheiden von Promotorverbindungen auf den Trägerteilchen gleichzeitig mit oder getrennt vom Abscheiden der Silberverbindungen.

21. Verwenden des Silberkatalysators nach den Ansprüchen 1 bis 4 zur Oxidation von Ethylen zu Ethylenoxid mittels Sauerstoff in der Gasphase.

## Claims

1. A silver catalyst for the oxidation of ethylene to ethylene oxide, containing from 0.1 to 25 per cent by weight of silver, based on the weight of the finished catalyst, on a homogeneously porous, heat-resistant support having a BET surface area of less than 2 m²/g, and having a silver concentration which is non-uniform within a support particle and is significantly greater on the geometric surface and in the layer, less than 0.5 mm thick, close to the surface of a support particle than inside the support particle, with the silver particles predominantly having a diameter of from 0.01 to 1 µm, obtainable by
- selection of moderately soluble silver compounds and formulations which give, at 50°C, a solution containing less than 400 g of silver per litre of solution,
- adjustment of the silver concentration of the impregnation solution to a value at which the concentration of the silver compound is at least 10% below the saturation concentration,
- adjustment of the temperature during impregnation of the support to a value at which the silver compound remains in solution,
- pre-drying of the impregnated support at a temperature of from 40°C to 120°C,
- thermoforming of the support in a gas stream, with evacuation repeatedly being carried out during the impregnation and pre-drying.

2. A silver catalyst according to claim 1, characterized by
- a layer, less than 0.3 mm thick, close to the surface of the support particle having a significantly higher concentration of silver.

3. A silver catalyst according to either claim 1 or 2, characterized by
- promoters, present in addition to the silver, comprising sodium, potassium rubidium, caesium and/or barium compounds in a concentration of from 0.0005 to 0.03 gram-equivalent per kilogram of catalyst.

4. A silver catalyst according to any of claims 1 to 3, characterized by
- a porous, heat-resistant support which comprises alpha-aluminium oxide in a purity of greater than 80 per cent by weight.

5. A process for the preparation of a silver catalyst according to any of claims 1 to 4, in which a homogeneously porous, heat-resistant support is impregnated with a silver-containing solution, and the impregnated support is pre-dried and thermoformed, characterized by
- selection of moderately soluble silver compounds and formulations which give, at 50°C, a solution containing less than 400 g of silver per litre of solution,
- adjustment of the silver concentration of the impregnation solution to a value at which the concentration of the silver compound is at least 10% below the saturation concentration,
- adjustment of the temperature during impregnation of the support to a value at which the silver compound remains in solution,
- pre-drying of the impregnated support at a temperature of from 40°C to 120°C,
- thermoforming of the support in a gas stream, with evacuation repeatedly being carried out during the impregnation and pre-drying.

6. A process according to claim 5, characterized by
- selection of moderately soluble silver compounds and formulations which give, at 50°C, a solution containing less than 330g of silver per litre of solution,
- adjustment of the silver concentration of the impregnation solution to a value at which the concentration of the silver compound is at least 20% below the saturation concentration,
- adjustment of the temperature during impregnation of the support to a value at which the silver compound remains in solution,
- pre-drying of the impregnated support at a temperature of from 60°C to 90°C,
- thermoforming of the support in a gas stream.

7. A process according to either claim 5 or 6, characterized by
- the use of water, or
- the use of an organic liquid or
- the use of a mixture of organic liquids as solvent for the silver-containing impregnation solution.

8. A process according to either claim 5 or 6, characterized by
- the use of a water-containing organic liquid as solvent for the silver-containing impregnation solution.

9. A process according to either claim 5 or 6, characterized by
- the use of ethanol as solvent for the silver-containing impregnation solution.

10. A process according to any of claims 5 to 9, characterized by
- selection of a silver solution in which the silver is in colloidal form.

11. A process according to any of claim 5 to 10, characterized by
- selection of a colloidal silver solution in which the silver colloid is protected by another colloid.

12. A process according to any of claims 5 to 11, characterized by
- pre-drying of the impregnated support by means of a gas stream passed through or over the support.

13. A process according to any of claims 5 to 12, characterized by
- pre-drying of the impregnated support at a reduced pressure which is above 20 kPa (200 mbar) absolute for at least 30 minutes.

14. A process according to any of claims 5 to 12, characterized by
- pre-drying of the impregnated support at a reduced pressure which is above 20 kPa (200 mbar) absolute for at least 60 minutes.

15. A process according to any of claims 5 to 14, characterized by
- repeated aeration and evacuation of the impregnation and drying vessel at intervals of from 1 to 30 minutes for from 1 to 30 minutes in each case.

16. A process according to any of claims 5 to 14, characterized by
- repeated aeration and evacuation of the impregnation and drying vessel at intervals of from 5 to 15 minutes for from 3 to 15 minutes in each case.

17. A process according to any of claims 5 to 16, characterized by
- addition of a small amount of initiators which favour the formation of crystal nuclei to the impregnation solution.

18. A process according to any of claims 5 to 16, characterized by
- addition of barium peroxide to the impregnation solution.

19. A process according to any of claims 5 to 18, characterized by
- forming of the pre-dried support particles in an agitated bed having a thickness which corresponds to from one to five times the smallest dimensions of a support particle, by means of a gas stream which predominantly comprises nitrogen with a maximum of 12 per cent by weight of oxygen, for from 5 to 240 minutes at a temperature of from 100 to 250°C.

20. A process according to any of claims 5 to 19, characterized by
- deposition of promoter compounds onto the support particles simultaneously with or separately from the deposition of the silver compounds.

21. Use of a silver catalyst according to any of claims 1 to 4 for the oxidation of ethylene to ethylene oxide by means of oxygen in the gas phase.

## Revendications

1. Catalyseur à l'argent pour l'oxydation de l'éthylène en oxyde d'éthylène, présentant une teneur en argent de 0,1 à 25 % en masse (relativement à la masse du catalyseur fini) sur un support à porosité homogène résistant à la chaleur, comportant une surface BET inférieure à 2 m²/g ayant une concentration non-uniforme de l'argent à l'intérieur d'une particule du support qui, sur la surface géométrique et dans la couche voisine de la surface d'une particule du support qui présente une épaisseur inférieure à 0,5 mm, est nettement plus grande qu'à l'intérieur de la particule du support, les particules d'argent présentant surtout un diamètre de 0,01 à 1 µm, que l'on peut obtenir par :
- le choix de composés d'argent modérément solubles et de formulations fournissant, à 50°C, une solution comportant moins de 400 g d'argent par litre de solution,
- l'ajustement de la concentration en argent de la solution d'imprégnation à une valeur pour laquelle la concentration du composé d'argent se situe à 10 % au moins en-dessous de la concentration de saturation,
- l'ajustement de la température, lors de l'imprégnation du support, à une température pour laquelle le composé d'argent reste en solution,
- le séchage préalable du support imprégné à une température de 40°C à 120°C,
- le façonnage thermique du support dans un courant gazeux.

2. Catalyseur à l'argent selon la revendication 1, caractérisé par :
- une couche voisine de la surface de la particule du support, qui est d'une épaisseur inférieure à 0,3 mm et présente une concentration en argent nettement plus grande.

3. Catalyseur à l'argent selon les revendications 1 et 2, caractérisé par :
- des promoteurs présents en plus de l'argent, constitués par des composés de sodium, de potassium, de rubidium, de césium et/ou de baryum, dans une concentration de 0,0005 à 0,03 gramme-équivalent par kilogramme de catalyseur.

4. Catalyseur à l'argent selon les revendications 1 à 3, caractérisé par :
- un support poreux résistant à la chaleur, qui est constitué par de l'oxyde d'alpha-aluminium d'une pureté supérieure à 80 % en masse.

5. Procédé de préparation d'un catalyseur à l'argent selon les revendications 1 à 4, dans lequel un support a porosité homogène résistant à la chaleur est imprégné avec une solution argentifère, le support imprégné étant soumis à un séchage préalable et à un façonnage thermique, caractérisé par :
- le choix de composés d'argent modérément solubles et de formulations fournissant, à 50°C, une solution comportant moins de 400 g d'argent par litre de solution,
- l'ajustement de la concentration en argent de la solution d'imprégnation à une valeur pour laquelle la concentration du composé d'argent se situe à 10 % au moins en-dessous de la concentration de saturation,
- l'ajustement de la température, lors de l'imprégnation du support, à une température pour laquelle le composé d'argent reste en solution,
- le séchage préalable du support imprégné à une température de 40°C à 120°C,
- le façonnage thermique du support dans un courant gazeux.

6. Procédé selon la revendication 5, caractérisé par :
- le choix de composés, modérément solubles, de l'argent et de formulations fournissant à 50°C une solution comportant moins de 330 g d'argent par litre de solution,
- l'ajustement de la concentration en argent de la solution d'imprégnation à une valeur pour laquelle la concentration du composé d'argent se situe au moins 20 % en-dessous de la concentration de saturation,
- l'ajustement de la température, lors de l'imprégnation du support, à une valeur pour laquelle le composé d'argent reste en solution,
- le séchage préalable du support imprégné à une température de 60°C à 90°C,
- le façonnage thermique du support dans un courant gazeux.

7. Procédé selon les revendications 5 et 6, caractérisé par :
- l'utilisation d'eau, ou
- l'utilisation d'un liquide organique, ou
- l'utilisation d'un mélange de liquides organiques en tant que solvants pour la solution d'imprégnation argentifère.

8. Procédé selon les revendications 5 et 6, caractérisé par
- l'utilisation d'un liquide organique aqueux en tant que solvant pour la solution d'imprégnation argentifère.

9. Procédé selon les revendications 5 et 6, caractérisé par :
- l'utilisation d'éthanol en tant que solvant pour la solution argentifère d'imprégnation.

10. Procédé selon les revendications 5 à 9, caractérisé par :
- le choix d'une solution d'argent dans laquelle l'argent se présente sous forme colloïdale.

11. Procédé selon les revendications 5 à 10, caractérisé par :
- le choix d'une solution colloïdale d'argent dans laquelle le colloïde d'argent est protégé par un autre colloïde.

12. Procédé selon les revendications 5 à 11, caractérisé par :
- le séchage préalable du support imprégné à l'aide d'un courant gazeux passant à travers ou au dessus.

13. Procédé selon les revendications 5 a 12, caractérisé par :
- le séchage préalable du support imprégné sous une pression réduite qui se situe au-dessus de 20 kPa (200 mbar) absolus, ce pendant 30 minutes au moins.

14. Procédé selon les revendications 5 à 12, caractérisé par :
- le séchage préalable du support imprégné sous une pression réduite qui se situe au-dessus de 20 kPa (200 mbar) absolus, ce pendant 60 minutes au moins.

15. Procédé selon les revendications 5 à 14, caractérisé par
- l'aérage répété et la mise sous vide du récipient d'imprégnation et de séchage à des intervalles de 1 a 30 minutes, chaque fois pendant une durée de 1 à 30 minutes.

16. Procédé selon les revendications 5 à 14 caractérisé par :
- l'aérage répété et la mise sous vide du récipient d'imprégnation et de séchage à des intervalles de 5 a 15 minutes, chaque fois pendant une durée de 3 a 15 minutes.

17. Procédé selon les revendications 5 à 16, caractérisé par :
- l'addition à la solution d'imprégnation d'une faible quantité d'initiateurs qui favorisent la formation de germes cristallins.

18. Procédé selon les revendications 5 à 16, caractérisé par :
- l'addition de peroxyde de baryum à la solution d'imprégnation.

19. Procédé selon les revendications 5 à 18, caractérisé par :
- le façonnage des particules préalablement séchées du support dans une couche mobile, avec une épaisseur de couche correspondant du simple ou quintuple des dimensions les plus petites d'une particule du support à l'aide d'un courant gazeux constitué surtout par de l'azote avec au maximum 12 % en masse d'oxygène, ce pendant 5 à 240 minutes à une température de 100 à 250°C.

20. Procédé selon les revendications 5 à 19, caractérisé par :
- la séparation des composés promoteurs sur les particules du support en même temps que ou séparément de la séparation des composés d'argent.

21. L'utilisation du catalyseur a l'argent selon les revendications 1 à 4 pour l'oxydation de l'éthylène en oxyde d'éthylène à l'aide d'oxygène en phase gazeuse.
